# EUROPEAN PATENT APPLICATION

(11) **EP 4 435 083 A1**
(43) Date of publication of application: **25.09.2024**
(21) Application number: 23164137.4
(22) Date of filing: 24.03.2023
(51) Int. Cl.: C12M 1/34, C12M 1/36

(54) **INTEGRATED BIOPROCESSING SYSTEM FOR PERFORMING SEVERAL BIOPROCESSES ON LIQUID IMMUNE OR NAIVE CELL CULTURES**

(71) Applicant: The Automation Partnership (Cambridge) Ltd., Hertfordshire SG8 5WY (GB)
(72) Inventor: Stacey, Adrian, Royston, Hertfordshire SG8 5WY (GB); Prouté, Fanny, Royston, Hertfordshire SG8 5WY (GB)
(74) Representative: Gottschald Patentanwälte Partnerschaft mbB

(57) **Abstract**

The invention relates to an integrated bioprocessing system for performing several bioprocesses on liquid immune or naive cell cultures, wherein the integrated bioprocessing system (1) performs several different bioprocesses by following different protocols, wherein the protocols define operations that the integrated bioprocessing system (1) performs on the cell culture of the respective bioprocess, wherein the integrated bioprocessing system (1) detects via a sensor (6) at least one state of the cell culture, wherein the protocols define reactions to be performed by the integrated bioprocessing system (1) in case of a deviation of the state of the cell culture from a normal state defined in the protocol, wherein the integrated bioprocessing system (1) detects, in particular detects via a sensor (6), at least one state of the integrated bioprocessing system (1). It is proposed that the protocols define reactions to be performed by the integrated bioprocessing system (1) in case of a deviation of the state of the integrated bioprocessing system (1) from a normal state defined in the protocol.

## Description

The present invention relates to an integrated bioprocessing system for performing several bioprocesses on liquid immune or naive cell cultures according to the general part of claim 1 and to a method for operating an integrated bioprocessing system according to the general part of claim 14.

The term "bioprocess" presently represents a biotechnological process, in particular a biotechnological process involving the use of immune cell cultures or naive cell cultures, in particular stem cell cultures. One or more processing steps might be performed on each cell culture. Hence, a bioprocess in this sense might refer to a manufacturing process that involves a sequence of processing steps performed on a cell culture which ultimately will lead to a final product.

Here, the bioprocess is in the area of cell and gene therapy, for example to manufacture autologous T cells that are modified to express a chimeric antigen receptor (CAR). These cells might be used for the treatment of various types of hematologic malignancies, including different types of leukemia (blood cancer). Other cell therapies based on naive cells, in particular stem cells and their derivates are also of interest.

For biotechnological processes involving the use of liquid immune or naive cell cultures the starting material usually is quite heterogeneous regarding its composition, for example as each donor or patient may be in a different condition (e.g., in terms of disease progression, genetic make-up or the immune system history). Therefore, certain steps of the bioprocess need to be flexibly adapted and tailored to each individual cell culture. Additionally, each cell culture may respond differently to a certain processing step applied, which requires individual adjustments within the processing step such as amounts of reagents added or the duration of incubation steps.

Hence, depending on the features of the cell culture and the bioprocess to be performed, various parameters, including for example the type, sequence or configuration of processing steps performed on each immune cell culture will need to be adjusted to the individual features of the cell culture.

In bioprocesses in the area of cell and gene therapy with allogenic or autologous production of genetically modified immune cells, compliance of the bioprocess with regulatory demands is important and the bioprocess is typically highly regulated and needs to be approved by regulatory authorities.

To ensure the safety of the product, a quality by design approach, which aims to ensure that all sources of variability affecting the bioprocess are identified, explained and managed by appropriate measures, is typically followed. This includes that all aspects related to the bioprocess are understood and controlled as much as possible. Especially, those aspects that are critical for the quality of the product need to be identified and their impact, including any deviations from their set-points, on the product need to be assessed. In the resulting design space, deviations need to be managed to keep the bioprocess compliant with the approved bioprocess. Accordingly, process data, including the type and quantity of materials used, process conditions and any deviations must be documented, and appropriate actions need to be taken in case a deviation occurs.

Recently, more and more integrated bioprocessing systems have been proposed. Such integrated bioprocessing systems allow simultaneous performance of several bioprocesses on different cell cultures, wherein the processing steps to be performed can be adjusted according to the respective cell culture. By providing a protocol comprising a workflow for the respective bioprocesses to the integrated bioprocessing system, an automation of many unit operations becomes possible.

The known prior art (WO 2017/221155 A1) that builds the basis of the invention is related to an integrated bioprocessing system according to the general part of claim 1.

The known integrated bioprocessing system has a control unit that is able to react to certain states of the cell culture and making decisions on how to proceed further. If the system is used for a GMP process, it is probable that the control unit will have strong boundaries imposed on the decisions it can make. It is a challenge to improve the known integrated bioprocessing system such that on the one hand, the quality of decisions made, whether by the system or a user, is very high and on the other hand, more decisions can be made by the integrated bioprocessing system without needing user intervention.

The invention is based on the problem of improving the known integrated bioprocessing system such that a further optimization regarding the named challenge is reached.

The above-noted object is solved by the features of the characterizing part of claim 1.

Usually and without necessarily looking at bioprocesses, a system handling different protocols for different products might have reactions to some deviations of the production of the product coded into the protocol. For example, if the product fails a quality test, the product may be discarded. Reactions to deviations of states of the system, for example a malfunction of a transport device or of a functional device for the production of the product would usually be handled by the system itself, independently of the protocol. For example, if a transport device removing products from a production station fails, the production station may be halted such that products do not accumulate.

The main realization of the present invention is that the above is not transferable 1:1 into an integrated bioprocessing system. Handling of a cell culture cannot be halted without knowledge about the consequences for the cell culture. The standard fallback for such a case would be to involve an operator. However, always involving an operator is inefficient.

The invention is therefore based on the realization that the best response to a deviation in a state of the integrated bioprocessing system depends on the context within the protocol. For example, if a deviation in O₂ control occurs at the start of a step of culturing the cell culture, a transfer to another incubator may be the correct reaction, but if it occurs at the end of culture when the cell count is high enough to harvest, then harvesting straight away may be the preferred reaction.

Further, if for example a protocol requires the growth of a high density of cells and there is a drop in O₂ control, then responding by moving the cells to another incubator might be necessary even in response to a small deviation, but in the case of a protocol which only performs low density growth, then the response can be slower because the oxygen demand is lower.

All these different reactions may respond to the same state of the integrated bioprocessing system, namely a failure of the O₂ control system or the incubator. The "correct" or preferred reaction however depends on the protocol.

It is therefore proposed to include reactions to states of the integrated bioprocessing system in the protocol. It is then possible to react to the same deviation of the integrated bioprocessing system in different ways depending on the protocol. Therefore, the decision by the integrated bioprocessing system as to what to do can depend on both the cell culture parameters monitored and the state of the integrated bioprocessing system, as well as the progress of execution of the protocol and some history of the cell culture like past measurements of the integrated bioprocessing system relating to the cell culture.

A further advantage comes from the fact that the cell culture state will typically lag behind any deviation in the state of the integrated bioprocessing system. If there is a fault with temperature control for example, the cell counts at the time when the fault occurs will be normal, and may remain normal for some time (e.g. hours); but with the proposed solution, the integrated bioprocessing system can handle the deviation before the cell culture itself enters an observably deviating state.

Further, a protocol may pass regulatory approval while a mechanism in the integrated bioprocessing system automatically dealing with deviations of the integrated bioprocessing system and therefore affecting different protocols would be hard to approve as the consequences can't be known fully.

The invention relates to an integrated bioprocessing system for performing several bioprocesses on liquid immune or naive cell cultures, wherein the integrated bioprocessing system performs several different bioprocesses by following different protocols, wherein the protocols define operations that the integrated bioprocessing system performs on the cell culture of the respective bioprocess, wherein the integrated bioprocessing system detects via a sensor at least one state of the cell culture, wherein the protocols define reactions to be performed by the integrated bioprocessing system in case of a deviation of the state of the cell culture from a normal state defined in the protocol, wherein the integrated bioprocessing system detects, in particular detects via a sensor, at least one state of the integrated bioprocessing system.

The term "state of the integrated bioprocessing system" is to be understood as comprising any functional, physical and/or software state of the integrated bioprocessing system that is not measured as a state of the cell culture. Any measurement directly targeting the cell culture, for example also a measurement of a temperature in direct vicinity to the cell culture, for example by a temperature sensor attached to a receptacle containing the cell culture and moving together with the cell culture, is not a measurement of a state of the integrated bioprocessing system. A general temperature measurement of an incubation chamber which would for example indicate that the incubation chamber has a defect and which influences the cell culture with a certain delay on the other hand is a measurement of a state of the integrated bioprocessing system. Any state that is inseparably a state of the cell culture and the integrated bioprocessing system is per definition a state of the cell culture and not of the integrated bioprocessing system. Here and preferably, the state of the integrated bioprocessing system is detected by a sensor.

In detail, it is proposed that the protocols define reactions to be performed by the integrated bioprocessing system in case of a deviation of the state of the integrated bioprocessing system from a normal state defined in the protocol.

In a very preferred embodiment according to claim 2 at least one protocol covers the whole measurement space of a sensor. Preferably, this is the case for multiple or all sensors. Therefore, not only singular states of the integrated bioprocessing system or certain trigger events are covered.

The progress of a protocol or an operation may have an influence on the reaction as described with the O₂ control example above (claim 3).

Claim 4 clarifies that different protocols may contain different reactions to the same state of the integrated bioprocessing system.

Embodiments according to claims 5 relate to partitioning the measurement space of a sensor to cover the whole measurement space. Some partitions may be linked to user actions according to claim 6.

In a preferred embodiment according to claim 7, the user actions may be stored. Also, the partitioning may be adaptable by the integrated bioprocessing system by learning from the user actions. The stored data or the automatic adaption allow improving the protocols over time.

Claim 8 describes that states of the integrated bioprocessing system may be and/or may not be coupled to the cell culture. Claims 9 and 10 give preferred embodiments of such states.

In a preferred embodiment according to claim 11, the whole measurement space of the integrated bioprocessing system may be covered, making the protocol the main or even only source of reactions to deviations. The complexity of this solution can be managed by originally mapping all states or almost all states of a new protocol to a user intervention and then improving the protocol over time.

According to claim 12 it may be the case that the integrated bioprocessing system reacts to a deviation of the integrated bioprocessing system before this deviation critically influences the cell culture.

In a preferred embodiment according to claim 13, in the case that a deviation concerns multiple cell cultures, which may be following different protocols, the integrated bioprocessing system prioritizes the reactions. If a transport mechanism for example fails, all cell cultures may be affected. Some may need immediate care while others may be able to remain even for days in an incubator or the like without problems.

Another teaching according to claim 14, which is of equal importance, relates to a method for operating an integrated bioprocessing system.

Here, it is essential that the protocols define reactions to be performed by the integrated bioprocessing system in case of a deviation of the state of the integrated bioprocessing system from a normal state defined in the protocol.

All explanations given with regard to the proposed integrated bioprocessing system are fully applicable.

Claim 15 relates to a method of adapting the protocol over time.

In the following, embodiments of the invention are explained with respect to the drawing. The drawing shows in
- Fig. 1,: an integrated bioprocessing system performing several bioprocesses on cell cultures and
- Fig. 2,: a snapshot in time showing testing for deviations and possible reactions.

The integrated bioprocessing system 1 shown in the drawing is preferably adapted to perform a bioprocess for the manufacturing of genetically modified T cells. Here, T cells are genetically modified to express a chimeric antigen receptor (CAR). Consequently, the term "CAR-T cells" describes T cells that have been genetically modified to express a CAR. The genetically modified CAR-T cells, which represent the product of the bioprocess, may be administered to a patient and used to start or resume cancer treatment in the patient. As the bioprocess is performed, the initial immune cell culture is gradually processed. All explanations given are mainly directed to such a bioprocess. It may be pointed out, however, that those explanations are fully applicable to other bioprocesses as well.

The term "liquid immune cell culture" is to be understood in a broad sense and refers to an immune cell culture comprising at least one type of immune cells suspended as particles in any type of liquid. As will be explained below, the liquid immune cell culture may comprise other cell types that are not immune cells. Hence, the term "liquid immune cell culture" refers to a liquid immune cell culture at any stage during the bioprocess. Consequently, the type and fraction of immune cells present in the liquid immune cell culture will change during the bioprocess applied as certain immune cells are enriched or depleted from the liquid immune cell culture and/or the immune cells are genetically modified.

The term "immune cells" generally refers to different types of white blood cells. Hence, the term "immune cells" includes a variety of cells, for example, but not limited to dendritic cells, T lymphocytes, also referred to as T cells, B lymphocytes, natural killer cells, macrophages or the like. Immune cells may also include subtypes of immune cells, for example tumor-infiltrating lymphocytes or different types of T cells. Subtypes of a certain type of immune cells may be classified based on the type of antigen present at the cell surface. Hence, the term immune cells may for example refer to T cells comprising the surface antigen CD4 ("CD4+ T cells"). Typically, a certain type of immune cells, e.g., T cells, preferably a certain subtype of immune cells, e.g., CD4+ T cells, will be selectively enriched by the bioprocess, while other immune cells, e.g. macrophages, and/or other cell types that are not immune cells, e.g., erythrocytes, and/or other subtypes of immune cells, e.g., CD8+ T cells, will be depleted from the liquid immune cell culture. The immune cells to be enriched are referred to as target immune cells, all other components to be depleted from the liquid immune cell culture are referred to "impurities". Further, and as mentioned above, the target immune cells might be genetically modified.

The term "naive cells" refers to cells that can still differentiate into different target cell types. In particular, stem cells and their derivatives prior to full differentiation into a specific cell type are naive cells. The term also comprises naive immune cells.

The term "liquid" is to be understood in a broad sense as well and refers to any liquid and/or particle-containing liquid that is processed within the integrated bioprocessing system 1. Hence, the term liquid might refer to media, waste, the liquid immune cell culture, byproducts obtained during the bioprocess, samples and/or an initial immune cell culture.

Preferably, an initial immune cell culture is obtained in a process called "leukapharesis". In leukapharesis, immune cells are obtained from the patient. Additionally or alternatively, the initial immune cell culture might also be obtained from a tissue of the patient. The initial immune cell culture might also be obtained by one or more donors that are not the patient.

Depending on the source of the initial cell culture and the bioprocess to be carried out, the initial cell culture, particularly the type, amount and distribution of impurities as well as target immune or naive cells might vary.

Presently, it is preferred that any initial immune cell culture used in the bioprocess is used for only a single patient, preferably the patient from which the cell culture was initially derived, or a small number of patients, for example up to ten patients. If that is not the case then the bioprocess presently described will yield only a cell culture for a single patient or a small number of patients and cell cultures for other patients are derived from different bioprocesses. Therefore, the proposed integrated bioprocessing system 1 is used for parallelizing small scale bioprocesses. Here and preferably, the bioprocesses are performed according to GMP.

Proposed is an integrated bioprocessing system 1 for performing several bioprocesses on liquid immune or naive cell cultures. The integrated bioprocessing system 1 performs the bioprocesses by controlling several components of the integrated bioprocessing system 1 to perform several unit operations. The integrated bioprocessing system 1 may comprise a transport mechanism 2 to transport the cell cultures between different stations for the unit operations. The integrated bioprocessing system 1 here and preferably keeps the cell cultures in closed environments, one for each cell culture. The closed environment may be adapted over the course of a bioprocess for example by tube welding. It is then not necessary to sterilize the whole system or use a clean room for the integrated bioprocessing system 1. Here and preferably, the integrated bioprocessing system 1 comprises a first interface 3 at which the cell culture can be entered into the integrated bioprocessing system 1 and/or a second interface 4 at which the cell culture leaves the integrated bioprocessing system 1. The integrated bioprocessing system 1 transports the cell culture from the first interface 3 to at least one, preferably multiple, positions at which unit operations are performed and preferably to the second interface 4 afterwards. Here and preferably, no user intervention is necessary and/or planned and/or performed between the first and the second interface 4 in a normal operation. The integrated bioprocessing system 1 may comprise an enclosure 5 protecting the space functionally between the interfaces from user interventions during a normal operation. The first and the second interface 4 may be the same interface.

Unit operations are basic process steps for reaching a certain physical and/or chemical change of the cell culture. Examples for common unit operations are enrichment, selection, activation and genetic modification.

The integrated bioprocessing system 1 performs several different bioprocesses by following different protocols. Exemplarily, a protocol may describe all steps from the cell culture entering the integrated bioprocessing system 1 to the cell culture leaving the integrated bioprocessing system 1.

The protocols define operations that the integrated bioprocessing system 1 performs on the cell culture of the respective bioprocess. The protocols may also describe further operations like transport operations, operations on additional media and the like. The operations may comprise unit operations, transport operations and so on.

The integrated bioprocessing system 1 detects via a sensor 6 at least one state of the cell culture. The integrated bioprocessing system 1 may comprise a sensor 6 for sensing the state of the cell culture. The integrated bioprocessing system 1 may comprise a mechanism for drawing samples from the cell culture and analyzing the samples.

The protocols define reactions to be performed by the integrated bioprocessing system 1 in case of a deviation of the state of the cell culture from a normal state defined in the protocol. Exemplarily, if a cell count is too low at a certain point, a further incubation may be performed.

The integrated bioprocessing system 1 further detects, in particular detects via a sensor 6, at least one state of the integrated bioprocessing system 1.

The states of the cell culture may comprise parameters of the cell culture like a cell count, a cell size distribution, a viability of the cell culture, a temperature of the cell culture, a density of the cell culture and the like. The parameters of the cell culture may include measurements taken of media containing the cells and/or of media removed from the cell culture.

The states of the integrated bioprocessing system 1 may comprise states like a malfunction of the transport mechanism 2, a general outage of an external power supply, a malfunction of an incubator, missing supplies and the like. Here and preferably at least one of the states of the integrated bioprocessing system 1 is not a temperature.

Essential is that the protocols define reactions to be performed by the integrated bioprocessing system 1 in case of a deviation of the state of the integrated bioprocessing system 1 from a normal state defined in the protocol.

Reactions are operations performed in reaction to deviations. The normal state does not have to be explicitly declared as such. A deviation leads to a path in a protocol that would not lead to a successful completion of the bioprocess without the reaction or otherwise would lead to a suboptimal result.

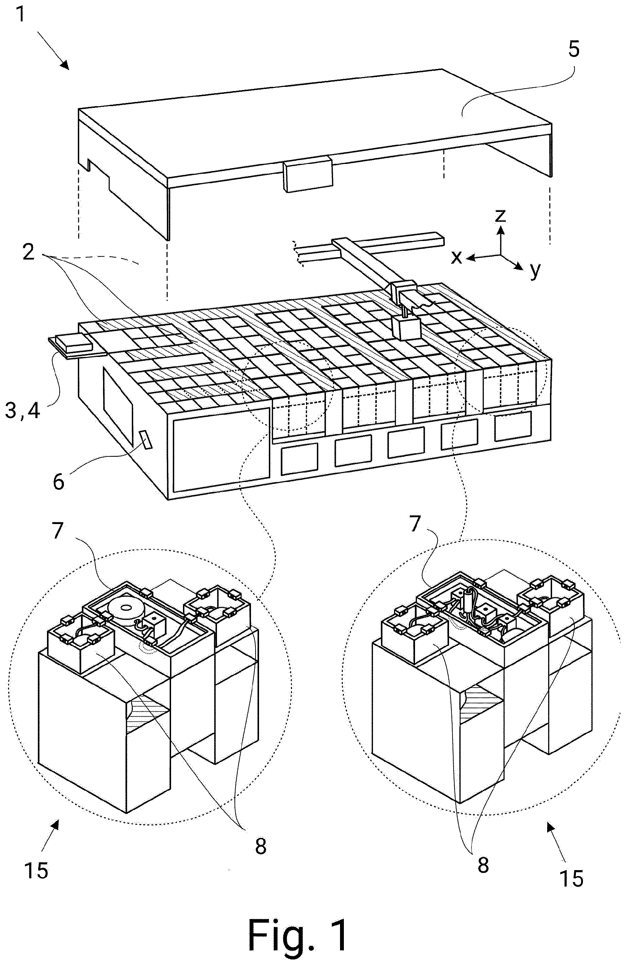

Fig. 1 shows an integrated bioprocessing system 1. Exemplarily, two bioprocesses being performed are shown in enlarged views. In detail, the enlarged views show each a cartridge 7 with a cell culture in which a unit operation is being performed connected to two receptacles 8 for transporting media and/or the cell culture through the integrated bioprocessing system 1. If none of the deviations is present, both protocols keep running through the normal path 9 along the normal states.

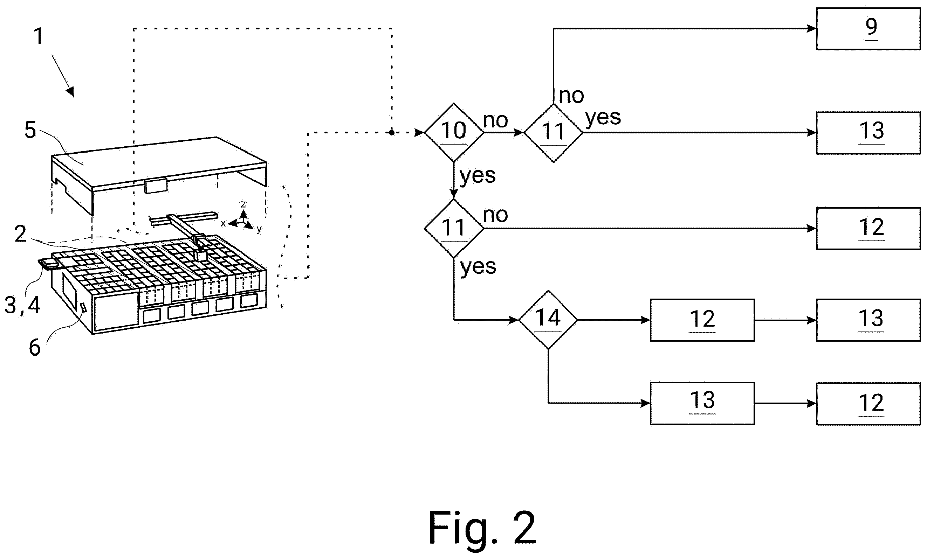

Fig. 2 shows a snapshot in time with decisions for the two bioprocesses. From left to right Fig. 2 shows that at a certain point in time the integrated bioprocessing system 1 analyses sensor 6 readings. The dotted lines show a line for the states of the cell cultures and a line for the states of the integrated bioprocessing systems 1. Exemplarily, the integrated bioprocessing system 1 tests for a first deviation 10 in the protocol of the first of the two bioprocesses and a second deviation 11 in the protocol of the second of the two bioprocesses. If only one of the deviations is present, the integrated bioprocessing system 1 executes a reaction of the first protocol 12 or a reaction of the second protocol 13.

If however both protocols have a deviation and exemplarily in the case that both deviations cannot be handled at the same time, the integrated bioprocessing system 1 enters a prioritization routine 14 and prioritizes between the deviations based on information from the protocols.

Here and preferably, at least one protocol defines, in particular multiple protocols define, a reaction of the integrated bioprocessing system 1 for part of a, in particular the whole, measurement space of at least one sensor 6, in particular of multiple sensors 6 per protocol. The measurement space of a sensor 6 comprises all possible values of the sensor 6. The measurement space may however be restricted to values that can actually occur. For example, the measurement space of a temperature sensor 6 does not have to comprise 0 K or 5000 K. By defining the measurement spaces of multiple sensors 6 in a protocol, many states of the integrated bioprocessing system 1 can be handled. Sensors 6 may comprise hardware and/or software sensors 6. The sensors 6 sensing states of the integrated bioprocessing system 1 here and preferably comprise or are sensors 6 that are permanently connected to the integrated bioprocessing system 1.

Here and preferably, said at least one sensor 6 is, in particular said multiple sensors 6 per protocol are, sensing a state or states of the integrated bioprocessing system 1. Generally, any time a sensor 6 is mentioned herein it is preferably a sensor 6 sensing a state of the integrated bioprocessing system 1 unless stated otherwise. Generally, any time "at least one" is mentioned, the term may in preferred embodiments be substituted by "at least two" or "at least three".

It may further be the case that at least one protocol defines reactions to the same state of the integrated bioprocessing system 1 differently dependent on the progress of the protocol. For example, a failure of the transport mechanism 2 may be handled differently if the cell culture is currently being transported, should be transported soon or doesn't have to be transported within the next day.

Preferably, at least one protocol defines reactions to the same state of the integrated bioprocessing system 1 differently dependent on the progress of an operation of the protocol. For example, the failure of the transport mechanism 2 can be handled differently during an incubation operation if the incubation just started or is about to end.

Consequently and in a preferred embodiment, different protocols may define different reactions to the same state of the integrated bioprocessing system 1, preferably even in the same context, more preferably even in the case that everything else is equal. For example, two protocols for immune cell therapy with similar cell cultures in the same incubator having entered the incubator at about the same time and staying in the incubator for about the same time and even having the same operations performed during the protocol and being at the same point in the protocol may define different reactions to a failure of the incubator. It may therefore also be the case that different protocols define different reactions to the same state of the integrated bioprocessing system 1 during an operation of the same type.

According to a preferred embodiment it is proposed that the measurement space of at least one of the sensors 6 and/or the measurement space of multiple sensors 6 is partitioned into at least two, preferably at least three, more preferably at least four, different partitions with different reactions. Partitioning a measurement space is a particularly easy way of handling the measurement space. For example, the temperature of the cell culture may need to stay between 35°C and 38°C according to the protocol. This may then be the normal partition. Further, partitions between 34° C and 35° C and between 38° C and 40° C may have associated reactions to be performed by the integrated bioprocessing system 1 like emergency cooling or heating. The remaining measurement space may be a partition with the associated reaction of immediately alerting an operator through a critical emergency routine, possibly highlighting the cell culture in the integrated bioprocessing system 1 or ejecting the cell culture from the integrated bioprocessing system 1 via an emergency interface.

Preferably, the measurement space of one and the same sensor 6 is partitioned into different partitions in different protocols and/or dependent on the progress of the protocol and/or dependent on the progress of an operation of the protocol. As will be explained, the partitioning of a protocol may change over time to better adapt the protocol to different possible states. Over time, the protocol may include a variety of reactions to different possible states, reducing the necessity of a user intervention.

According to one embodiment it is proposed, that at least one reaction, in particular of one partition, comprises informing a user and/or that at least one reaction, in particular of one partition, does not comprise informing a user.

After informing the user, the integrated bioprocessing system 1 may receive a reaction to be performed directly or indirectly from the user. The integrated bioprocessing system 1 performs the reaction and saves the received reaction in relation to the template and the state of the integrated bioprocessing system 1 that led to informing the user. The saved information can later be displayed to a user or can be used for analysis. The integrated bioprocessing system 1 may additionally or alternatively adapt the template based on the received reaction, in particular such that the same state does not lead to informing a user in the future.

It may be the case that the at least one state of the integrated bioprocessing system 1 comprises a state indirectly coupled to a state of the cell culture, and/or, that the at least one state of the integrated bioprocessing system 1 comprises a state not coupled to a state of the cell culture.

The state or states of the integrated bioprocessing system 1 indirectly coupled to the cell culture may include an energy input into the cell culture, in particular heating or cooling energy, and/or a volume or mass input into the cell culture, in particular a gas flow rate and/or a base and/or ammonium addition rate. The term "indirectly coupled" means that a state has an influence generally on the biological state of the cell culture, in particular by being a state describing the provision of media or energy.

The state or states of the integrated bioprocessing system 1 not coupled to the cell culture may include a state, in particular an availability, of a transport mechanism 2 of the integrated bioprocessing system 1 and/or a presence of a, in particular predefined, receptacle 8, in particular not containing the cell culture, and/or consumable at a, in particular predefined location, and/or a state of an operation station 15 of the integrated bioprocessing used to perform the bioprocess according to the template.

To summarize, states of the cell culture may comprise one or more of a cell density, in particular compared against a known trajectory, a cell type distribution, in particular compared against a desired distribution, a cell characteristic distribution, in particular a proportion of genetically modified cells, a pH value, a dissolved oxygen value, a rate of oxygen update, a rate of oxygen uptake, a glucose concentration, a carbon dioxide concentration, a rate of carbon dioxide evolution, a cell viability, an enzyme activity, a lactate concentration and/or an ammonium ion concentration.

The indirectly coupled states may comprise one or more of oxygen fed to a cell culture, heat fed to a cell culture, energy or volume fed to a cell culture. Here and preferably the indirectly coupled states have a time lag between a change of state of the integrated bioprocessing system 1 and a state of the cell culture. The time lag may be specified in the protocol.

The states not coupled may comprise states informing about a defect of a part of the integrated bioprocessing system 1 that does not currently influence the cell culture, for example a transport mechanism 2, here a transport robot, for transport of the cell culture to a next station.

According to one embodiment it is proposed, that in at least one protocol the whole measurement space of all sensors 6 of the integrated bioprocessing system 1 sensing states of the cell culture assigned to the protocol and preferably the whole measurement space of all sensors 6 of the integrated bioprocessing system 1 sensing states of the integrated bioprocessing system 1 coupled to the state of the cell culture and more preferably the whole measurement space of all sensors 6 of the integrated bioprocessing system 1 sensing states of the integrated bioprocessing system 1 related to the performance of the protocol is covered in the protocol. States of parts not used in the protocol do not have to be covered if independently of those states the protocol can be executed.

It is preferably the case that the reaction in case of a deviation of the state of the integrated bioprocessing system 1 from a normal state defined in the protocol is performed before the deviation leads to a deviation in the state of the cell culture. The lag mentioned above can be used to protect the cell culture in case of a system deviation. As the lag and the question of how quickly a deviation meaningfully affects the cell culture may depend on the protocol, the proposed solution allows the cell culture to be protected preemptively. Preferably, the reaction is performed before the deviation leads to any change in the state of the cell culture.

According to one embodiment it is proposed, that in case that a change of a state of the integrated bioprocessing system 1 affects multiple cell cultures, the integrated bioprocessing system 1 derives from the protocols of the cell cultures a prioritization for the reactions in the protocols and performs the reactions according to the prioritization. This embodiment is shown in Fig. 2 as mentioned above. If for example an incubator with several cell cultures inside fails, some cell cultures may need immediate attention while others may be able to sustain a prolonged period in the incubator without further measures. The integrated bioprocessing system 1 may then for example decide the order of transferring the cell cultures out of the incubator based on the prioritization. The prioritization may be based on the time lag between the change of the state of the integrated bioprocessing system and changes of states of the cell cultures of different protocols.

Another teaching which is of equal importance relates to a method for operating an integrated bioprocessing system 1 performing several bioprocesses on liquid immune or naive cell cultures, wherein the integrated bioprocessing system 1 performs several different bioprocesses by following different protocols, wherein the protocols define operations that the integrated bioprocessing system 1 performs on the cell culture of the respective bioprocess, wherein the integrated bioprocessing system 1 detects via a sensor 6 at least one state of the cell culture, wherein the protocols define reactions to be performed by the integrated bioprocessing system 1 in case of a deviation of the state of the cell culture from a normal state defined in the protocol, wherein the integrated bioprocessing system 1 detects, in particular detects via a sensor 6, at least one state of the integrated bioprocessing system 1.

Essential according to this further teaching is that the protocols define reactions to be performed by the integrated bioprocessing system 1 in case of a deviation of the state of the integrated bioprocessing system 1 from a normal state defined in the protocol.

All explanations given with regard to the proposed integrated bioprocessing system 1 are fully applicable.

According to one embodiment it is proposed, that at least one protocol is adapted over time such that the number of partitions increases and/or the reactions in partitions change, in particular such that less partitions require informing a user, preferably, that the protocol is adapted at least partially automatically, and/or, that the integrated bioprocessing system 1 stores information about actions by a user in relation to the protocol requiring the user actions and that the stored information is output to a user for adapting the protocol.

The change may take two importantly different forms, on the one hand responses of the integrated bioprocessing system 1 may become more granular, i.e. the measurement space may be partitioned more and more into progressively better attuned responses and on the other hand responses of the integrated bioprocessing system 1 to particular states may be updated, for example if there is evidence that there is a better way of recovering from a deviation than the way in the protocol. One or both types of changes may be implemented.

## Claims

1. Integrated bioprocessing system for performing several bioprocesses on liquid immune or naive cell cultures, wherein the integrated bioprocessing system (1) performs several different bioprocesses by following different protocols, wherein the protocols define operations that the integrated bioprocessing system (1) performs on the cell culture of the respective bioprocess,
wherein the integrated bioprocessing system (1) detects via a sensor (6)at least one state of the cell culture,
wherein the protocols define reactions to be performed by the integrated bioprocessing system (1) in case of a deviation of the state of the cell culture from a normal state defined in the protocol,
wherein the integrated bioprocessing system (1) detects, in particular detects via a sensor (6), at least one state of the integrated bioprocessing system (1), **characterized in**
**that** the protocols define reactions to be performed by the integrated bioprocessing system (1) in case of a deviation of the state of the integrated bioprocessing system (1) from a normal state defined in the protocol.

2. Integrated bioprocessing system according to claim 1, **characterized in that** at least one protocol defines, in particular multiple protocols define, a reaction of the integrated bioprocessing system (1) for part of a, in particular the whole, measurement space of at least one sensor (6), in particular of multiple sensors (6) per protocol, preferably, that the at least one sensor (6) is, in particular the multiple sensors (6) per protocol are, sensing a state or states of the integrated bioprocessing system (1).

3. Integrated bioprocessing system according to claim 1 or 2, **characterized in that** at least one protocol defines reactions to the same state of the integrated bioprocessing system (1) differently dependent on the progress of the protocol, preferably, that at least one protocol defines reactions to the same state of the integrated bioprocessing system (1) differently dependent on the progress of an operation of the protocol.

4. Integrated bioprocessing system according to one of the preceding claims, **characterized in that** different protocols define different reactions to the same state of the integrated bioprocessing system (1), preferably, that different protocols define different reactions to the same state of the integrated bioprocessing system (1) during an operation of the same type.

5. Integrated bioprocessing system according to one of claims 2 to 4, **characterized in that** the measurement space of at least one of the sensors (6) and/or the measurement space of multiple sensors (6) is partitioned into at least two, preferably at least three, more preferably at least four, different partitions with different reactions, preferably, that the measurement space of one and the same sensor (6) is partitioned into different partitions in different protocols and/or dependent on the progress of the protocol and/or dependent on the progress of an operation of the protocol.

6. Integrated bioprocessing system according to one of the preceding claims, **characterized in that** at least one reaction, in particular of one partition, comprises informing a user and/or that at least one reaction, in particular of one partition, does not comprise informing a user

7. Integrated bioprocessing system according to claim 6, **characterized in that** the integrated bioprocessing system (1) receives a reaction to be performed after informing the user, that the integrated bioprocessing system (1) performs the reaction, that the integrated bioprocessing system (1) saves the received reaction in relation to the template and the state of the integrated bioprocessing system (1) that led to informing the user, and/or, that the integrated bioprocessing system (1) adapts the template based on the received reaction, in particular such that the same state does not lead to informing a user in the future.

8. Integrated bioprocessing system according to one of the preceding claims, **characterized in that** the at least one state of the integrated bioprocessing system (1) comprises a state indirectly coupled to a state of the cell culture, and/or, that the at least one state of the integrated bioprocessing system (1) comprises a state not coupled to a state of the cell culture.

9. Integrated bioprocessing system according to claim 8, **characterized in that** the state or states of the integrated bioprocessing system (1) indirectly coupled to the cell culture include an energy input into the cell culture, in particular heating or cooling energy, and/or a volume or mass input into the cell culture, in particular a gas flow rate.

10. Integrated bioprocessing system according to claim 8 or 9, **characterized in that** the state or states of the integrated bioprocessing system (1) not coupled to the cell culture include a state, in particular an availability, of a transport mechanism (2) of the integrated bioprocessing system (1) and/or a presence of a, in particular predefined, receptacle (8), in particular not containing the cell culture, and/or consumable at a, in particular predefined location, and/or a state of an operation station (15) of the integrated bioprocessing used to perform the bioprocess according to the template.

11. Integrated bioprocessing system according to one of the preceding claims, **characterized in that** in at least one protocol the whole measurement space of all sensors (6) of the integrated bioprocessing system (1) sensing states of the cell culture assigned to the protocol and preferably the whole measurement space of all sensors (6) of the integrated bioprocessing system (1) sensing states of the integrated bioprocessing system (1) coupled to the state of the cell culture and more preferably the whole measurement space of all sensors (6) of the integrated bioprocessing system (1) sensing states of the integrated bioprocessing system (1) related to the performance of the protocol is covered in the protocol.

12. Integrated bioprocessing system according to one of the preceding claims, **characterized in that** the reaction in case of a deviation of the state of the integrated bioprocessing system (1) from a normal state defined in the protocol is performed before the deviation leads to a deviation in the state of the cell culture, preferably before the deviation leads to any change in the state of the cell culture.

13. Integrated bioprocessing system according to one of the preceding claims, **characterized in that** in case that a change of a state of the integrated bioprocessing system (1) affects multiple cell cultures, the integrated bioprocessing system (1) derives from the protocols of the cell cultures a prioritization for the reactions in the protocols and performs the reactions according to the prioritization.

14. Method for operating an integrated bioprocessing system (1) performing several bioprocesses on liquid immune or naive cell cultures, wherein the integrated bioprocessing system (1) performs several different bioprocesses by following different protocols, wherein the protocols define operations that the integrated bioprocessing system (1) performs on the cell culture of the respective bioprocess,
wherein the integrated bioprocessing system (1) detects via a sensor (6)at least one state of the cell culture,
wherein the protocols define reactions to be performed by the integrated bioprocessing system (1) in case of a deviation of the state of the cell culture from a normal state defined in the protocol,
wherein the integrated bioprocessing system (1) detects, in particular detects via a sensor (6), at least one state of the integrated bioprocessing system (1), **characterized in**
**that** the protocols define reactions to be performed by the integrated bioprocessing system (1) in case of a deviation of the state of the integrated bioprocessing system (1) from a normal state defined in the protocol.

15. Method according to claim 14, **characterized in that** at least one protocol is adapted over time such that the number of partitions increases and/or the reactions in partitions change, in particular such that less partitions require informing a user, preferably, that the protocol is adapted at least partially automatically, and/or, that the integrated bioprocessing system (1) stores information about actions by a user in relation to the protocol requiring the user actions and that the stored information is output to a user for adapting the protocol.
